# EUROPÄISCHE PATENTSCHRIFT

(11) **EP 2 340 796 B1**
(45) Veröffentlichungstag und Bekanntmachung des Hinweises auf die Patenterteilung: **21.09.2011**
(21) Anmeldenummer: 09016075.5
(22) Anmeldetag: 29.12.2009
(51) Int. Cl.: A61F 13/56, D04H 13/00

(54) **Verfahren zur Herstellung einer Materialbahn, aus der elastisch dehnbare Windelverschlusselemente ausstanzbar sind**
Method for producing a sheet of material from which elastically extendable nappy closers can be stamped
Procédé de fabrication d'une bande de matériau à partir de laquelle des éléments de fermeture pour couche sont découpés

(43) Veröffentlichungstag der Anmeldung: 06.07.2011
(73) Patentinhaber: Nordenia Deutschland Gronau GmbH, 48599 Gronau (DE)
(72) Erfinder: Schönbeck, Marcus, 33775 Versmold (DE)
(74) Vertreter: Albrecht, Rainer Harald

(56) Entgegenhaltungen:
- EP-A1- 1 690 464
- EP-A1- 1 900 512
- EP-A2- 1 541 106
- WO-A1-2006/063232

## Beschreibung

Die Erfindung betrifft ein Verfahren zur Herstellung einer Materialbahn, aus der elastisch dehnbare Windelverschlusselemente ausstanzbar sind. Die Windelverschlusselemente weisen Deckschichten aus Nonwoven sowie einen zwischen den Deckschichten einkaschierten elastischen Folienstreifen auf, der ein elastisch dehnbaren Abschnitt des Windelverschlusselementes bildet. Die Deckschichten sind in Dehnungsrichtung breiter als der einkaschierte Folienstreifen und an überstehenden Abschnitten, welche nicht elastische Anschlussbereiche bilden, miteinander verbunden. Die nicht elastischen Anschlussbereiche werden zur Befestigung an einer Windel sowie zum Anschluss eines Verschlussteils genutzt. Verschlussteil meint insbesondere ein Flächenelement mit Haken oder Schlaufen, die einen Teil eines Klettverschlusses bilden.

Aus DE 10 2004 035 042 A1 sind Windelverschlusselemente mit den beschriebenen Merkmalen sowie ein Verfahren zur Herstellung einer Materialbahn, aus der die beschriebenen Verschlusselemente ausgestanzt werden können, bekannt. Die Windelverschlusselemente können als Streifen ausgebildet sein oder die Form sogenannter Windelohren aufweisen, deren windelseitiger Anschlussbereich breiter ist als der Anschlussbereich zur Befestigung des Verschlussteils. Über das Verschlussteil eines Klettverschlusses werden große Kräfte auf das Windelverschlusselement übertragen. Für eine gleichmäßige Krafteinleitung in das Windelverschlusselement ist ein möglichst biegesteifer Anschlussbereich hoher Zugfestigkeit vorteilhaft. In Bezug auf ihre Anschlussbereiche sind die Windelverschlusselemente noch verbesserungsbedürftig.

Der Erfindung liegt die Aufgabe zugrunde, ein Verfahren zur Herstellung elastischer Windelverschlusselemente anzugeben, die einen biegesteifen und zugfesten Anschlussbereich für ein Verschlussteil aufweisen. Die Windelverschlusselemente sollen sich ferner möglichst kostengünstig fertigen lassen.

Gegenstand der Erfindung und Lösung dieser Aufgabe ist ein Verfahren nach Anspruch 1 zur Herstellung einer Materialbahn, aus der elastisch dehnbare Windelverschlusselemente ausstanzbar sind. Nach dem erfindungsgemäßen Verfahren werden parallele und zueinander beabstandete Folienstreifen aus einem elastisch dehnbaren Polymer in einer ersten Kaschierstation zwischen zwei Materialbahnen aus Nonwoven einkaschiert und die Bahnabschnitte zwischen den elastischen Folienstreifen miteinander verklebt. Das in der ersten Kaschierstation gefertigte Laminat wird einer zweiten Kaschierstation zugeführt, in der zugfeste, nicht elastische Materialstreifen außenseitig auf das Laminat aufkaschiert werden. Die nicht elastischen Materialstreifen werden in der zweiten Kaschierstation auf Bahnabschnitte des Laminats aufgebracht, in denen die aus Nonwoven bestehenden Schichten unmittelbar miteinander verklebt sind. Die Bahngeschwindigkeit, mit der das Laminat die zweite Kaschierstation durchläuft, wird so gesteuert, dass diese Bahngeschwindigkeit stets kleiner ist als die Geschwindigkeit, mit der die Materialbahn aus Nonwoven und die elastischen Folienstreifen in der ersten Kaschierstation während der Laminatbildung zusammengeführt werden.

Das erfindungsgemäße Verfahren ist ein zweistufiger Kaschierprozess. In einer ersten Stufe des Kaschierprozesses entsteht eine aus Nonwovenschichten und einkaschierten Folienstreifen bestehende Materialbahn, die quer zur Bahnrichtung abwechselnd elastisch dehnbare Zonen und nicht elastische Zonen aus Nonwoven aufweist. Die Materialbahn ist inhomogen, und zwar sowohl hinsichtlich der Schichtdicke als auch der Dehnungseigenschaften. Durch den Wechsel aus elastischen und weniger elastischen Bereichen entsteht das Problem, dass sich quer zur Bahnrichtung Verwerfungen in der Materialbahn bilden können. Das Verfahren ist so zu führen, dass die Materialbahn als ebene Bahn ohne Verzug und Verwerfungen der zweiten Kaschierstation zugeführt werden kann, in der nicht elastische Materialstreifen außenseitig auf das Laminat aufkaschiert werden. Einen wesentlichen Beitrag zur Lösung dieses Problems leistet das Merkmal, dass die Bahngeschwindigkeit, mit der das Laminat die zweite Kaschierstation durchläuft, so gesteuert wird, dass diese Bahngeschwindigkeit stets kleiner ist als die Geschwindigkeit, mit der die Materialbahn aus Nonwoven und die elastischen Folienstreifen in der ersten Kaschierstation während der Laminatbildung zusammengeführt werden. Die Bahnspannung wird auf diese Weise so weit reduziert, dass die Materialbahn ohne Verzug und ohne Welligkeiten der zweiten Kaschierstation zuführbar ist. Im Rahmen des erfindungsgemäßen Verfahrens sind aufwendige Messungen der Bahnspannung nicht erforderlich. Insbesondere entfällt eine aufwendige Regelung der Bahnspannung, wenn die Bahngeschwindigkeiten erfindungsgemäß aufeinander abgestimmt werden.

Die Bahngeschwindigkeit in der zweiten Kaschierstation und die Geschwindigkeit, mit der die Materialbahn aus Nonwoven und die elastischen Folienstreifen in der ersten Kaschierstation zusammengeführt werden, werden zweckmäßig so eingestellt, dass die Geschwindigkeiten in einem gegebenen konstanten Verhältnis stehen. Das Verhältnis kann empirisch ermittelt werden, wobei die Werte zweckmäßig so eingestellt werden, dass die Bahngeschwindigkeit in der zweiten Kaschierstation um 0,1 bis 1,0 % von der Geschwindigkeit, mit der die Materialbahnen aus Nonwoven und die elastischen Folienstreifen in der ersten Kaschierstation zusammengeführt werden, abweicht.

Vorteilhafte Ausgestaltungen des erfindungsgemäßen Verfahrens werden in den nachgeordneten Patentansprüchen beschrieben. Zweckmäßig werden in der zweiten Kaschierstation Streifen aus Nonwoven oder Folienstreifen aus einem nicht elastischen Polymer als Verstärkungsstreifen auf einer ersten Seite des Laminats aufkaschiert. In der zweiten Kaschierstation können zusätzlich auch Bänder mit Verschlusselementen auf der zweiten Seite des Laminats aufkaschiert werden.

Die außenseitig auf das Laminat aufkaschierten nicht elastischen Materialstreifen sind zweckmäßig breiter als die Bahnabschnitte zwischen den elastischen Folienstreifen, so dass sich randseitige Überlappungen mit den elastischen Abschnitten ergeben. Der Überlappungsbereich kann beispielsweise 2 mm bis 10 mm breit sein und trägt zu einer gleichmäßigen Kraftübertragung zwischen dem versteiften Anschlussbereich und dem angrenzenden elastisch dehnbaren Abschnitt der aus der Materialbahn ausgestanzten Windelverschlusselemente bei.

Zur Herstellung des in der ersten Kaschierstation gefertigten Laminats und zum Aufkaschieren der nicht elastischen Materialstreifen in der zweiten Kaschierstation können Hotmelt-Klebstoffe verwendet werden.

Im Folgenden wird die Erfindung anhand einer lediglich ein Ausführungsbeispiel darstellenden Zeichnung erläutert. Es zeigen schematisch:
- **Fig. 1**: einen Schnitt durch eine Materialbahn, aus der elastisch dehnbare Windelverschlusselemente ausstanzbar sind,
- **Fig. 2**: eine Draufsicht auf die Materialbahn mit Stanzkonturen der Windelverschlusselemente,
- **Fig. 3**: ein Verfahren zur Herstellung der in den Fig. 1 und 2 dargestellten Materialbahn.

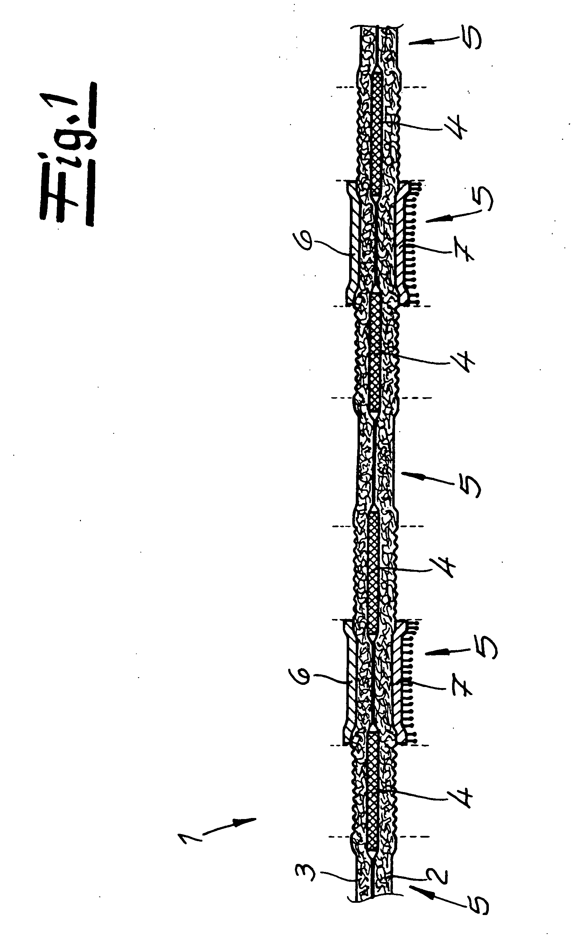

Die Fig. 1 zeigt einen Schnitt durch eine Materialbahn 1 quer zur Bahnlängsrichtung. Die Materialbahn 1 weist zwei Deckschichten 2, 3 aus Nonwoven und zwischen den Deckschichten 2, 3 einkaschierte Folienstreifen 4 aus einem elastisch dehnbaren Polymer auf. Die Bahnabschnitte 5 zwischen den elastischen Folienstreifen 4 sind miteinander verklebt. An der Außenseite der Materialbahn 1 sind auf einer ersten Seite des Laminats Verstärkungsstreifen 6 aufgebracht, die aus Nonwoven oder Folienstreifen aus einem nicht elastischen Polymer bestehen können. Auf der zweiten Seite des Laminats sind Bänder 7 mit Verschlusselementen aufgebracht. Bei den Verschlusselementen kann es sich um Haken oder Schlaufen, die beispielsweise durch Wirken erzeugt werden, handeln. Die Verstärkungsstreifen 6 sowie die Bänder 7 mit Verschlusselementen sind auf den Bahnabschnitten 5 des Laminats angeordnet, in denen die aus Nonwoven bestehenden Deckschichten 2, 3 unmittelbar miteinander verklebt sind. Der Darstellung in Fig. 1 entnimmt man auch, dass die an der Außenseite auf das Laminat aufkaschierten nicht elastische Materialstreifen 6, 7 breiter sind als die Bahnabschnitte 5 zwischen den elastischen Folienstreifen 4.

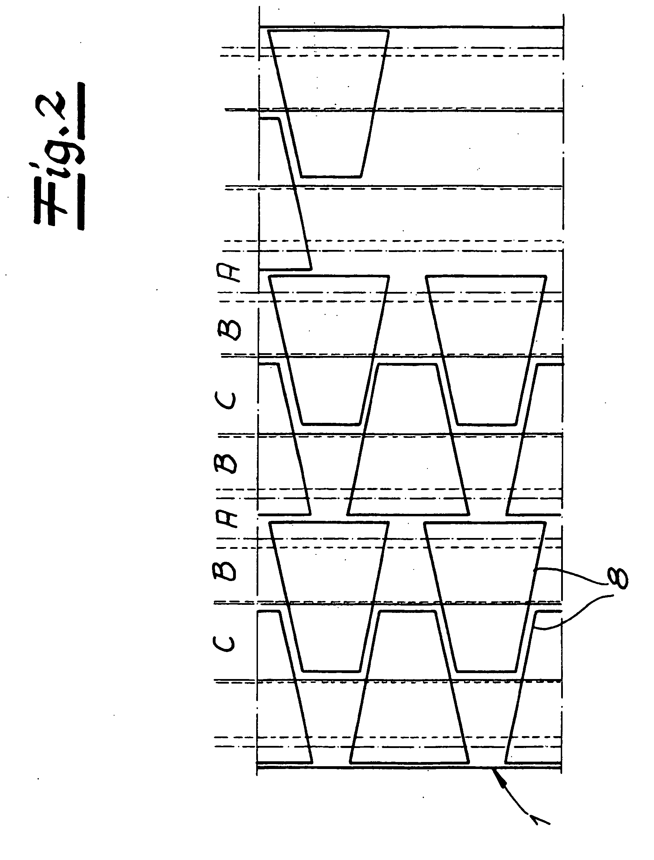

Die Fig. 2 zeigt eine Draufsicht auf das Laminat bzw. die mehrlagige Materialbahn 1. Das Ausführungsbeispiel zeigt eine mehmutzige Materialbahn 1, die quer zur Bahnrichtung eine sich mehrfach wiederholende Folge aus drei Zonen aufweist. In einer ersten Zone A sind die Deckschichten 2, 3 aus Nonwoven unmittelbar miteinander verbunden. Eine zweite Zone B ist elastisch dehnbar und weist Deckschichten aus Nonwoven 2, 3 sowie einen elastischen Folienstreifen 4 als Zwischenschicht auf. Die dritte Zone C besteht aus miteinander verklebten Nonwoven-Schichten 2, 3 sowie nicht elastische Materialstreifen 6, 7, die außenseitig auf das Laminat aufkaschiert worden sind und Verstärkungsstreifen aus einem nicht elastischen Polymer und/oder Bänder mit Verschlusselementen bilden. Aus der Materialbahn 1 können Windelverschlusselemente 8 ausgestanzt werden, deren Kontur in Fig. 2 zur Veranschaulichung dargestellt wurde. Bei den Windelverschlusselementen 8 handelt es sich beispielsweise um sogenannte Windelohren, deren windelseitiger Anschlussbereich in Maschinenlaufrichtung der Materialbahn 1 länger ist als der Anschlussbereich zur Befestigung eines Verschlussteils.

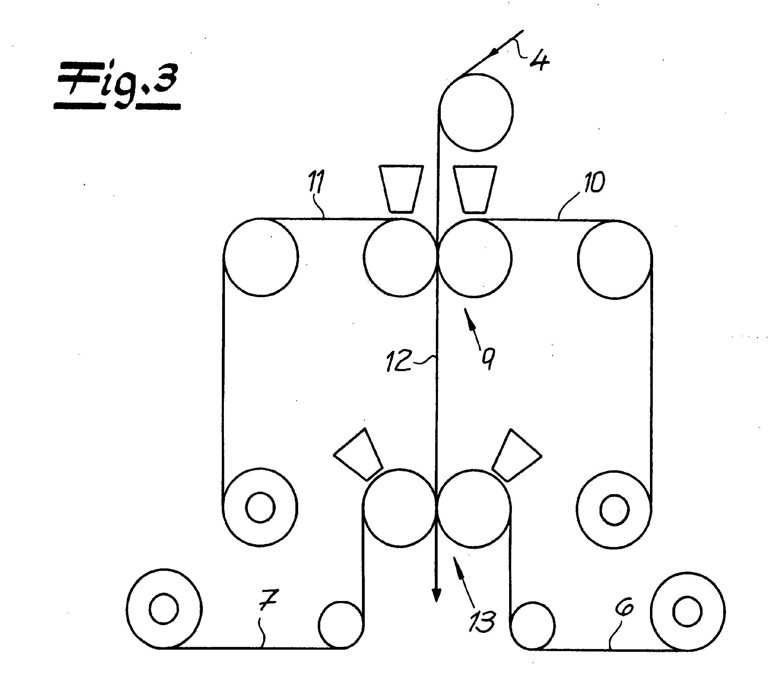

Das Verfahren zur Herstellung der beschriebenen Materialbahn ist in Fig. 3 dargestellt. In einer ersten Kaschierstation 9 werden parallele und zueinander beabstandete Folienstreifen 4 aus einem elastisch dehnbaren Polymer zwischen zwei Materialbahnen 10, 11 aus Nonwoven einkaschiert und werden die Bahnabschnitte zwischen den elastischen Folienstreifen 4 miteinander verklebt. Die Materialbahnen 10, 11 bilden die Deckschichten 2, 3 eines Laminates 12. Das in der Kaschierstation 9 gefertigte Laminat 12 wird einer zweiten Kaschierstation 13 zugeführt, in der zugfeste, nicht elastische Materialstreifen 6, 7 außenseitig auf das Laminat 12 aufkaschiert werden. Die nicht elastischen Materialstreifen 6, 7 werden in der zweiten Kaschierstation 13 auf Bahnabschnitte 5 des Laminats aufgebracht, in denen die aus Nonwoven bestehenden Schichten 10, 11 unmittelbar miteinander verklebt sind. Die Bahngeschwindigkeit, mit der das Laminat 12 die zweite Kaschierstation 13 durchläuft, wird erfindungsgemäß so gesteuert, dass diese Bahngeschwindigkeit stets kleiner ist als die Geschwindigkeit, mit der die Materialbahn 10, 11 aus Nonwoven und die elastischen Folienstreifen 4 in der ersten Kaschierstation 9 während der Laminatbildung zusammengeführt werden. Die Bahngeschwindigkeit in der zweiten Kaschierstation 13 und die Bahngeschwindigkeit in der ersten Kaschierstation 9 stehen in einem vorgegebenen konstanten Verhältnis zueinander. Das Verhältnis wird vorzugsweise so gewählt, dass die Bahngeschwindigkeit in der zweiten Kaschierstation 13 um 0,1 % bis 1,0 % von der Geschwindigkeit, mit der die Materialbahnen 10, 11 aus Nonwoven und die elastischen Folienstreifen 4 in der ersten Kaschierstation 9 zusammengeführt werden, abweicht. Durch die beschriebene Abstimmung der Bahngeschwindigkeiten kann die Bahnspannung vor der zweiten Kaschierstation 13 so weit reduziert werden, dass sich in der Materialbahn 1 keine Welligkeiten und kein Verzug einstellt.

Zur Herstellung des in der ersten Kaschierstation 9 gefertigten Laminats 12 und zum Aufkaschieren der nicht elastischen Materialstreifen 6, 7 in der zweiten Kaschierstation 13 werden Hotmelt-Klebstoffe verwendet, die in Klebestationen aufgebracht werden.

## Patentansprüche

1. Verfahren zur Herstellung einer Materialbahn, aus der elastisch dehnbare Windelverschlusselemente ausstanzbar sind,
wobei parallele und zueinander beabstandete Folienstreifen (4) aus einem elastisch dehnbaren Polymer in einer ersten Kaschierstation (9) zwischen zwei Materialbahnen (10, 11) aus Nonwoven einkaschiert und die Bahnabschnitte (5) zwischen den elastischen Folienstreifen (4) miteinander verklebt werden,
wobei das in der ersten Kaschierstation (9) gefertigte Laminat (12) einer zweiten Kaschierstation (13) zugeführt wird, in der zugfeste, nicht elastische Materialstreifen (6, 7) außenseitig auf das Laminat (12) aufkaschiert werden,
wobei die nicht elastischen Materialstreifen (6, 7) auf Bahnabschnitte des Laminats (12) aufgebracht werden, in denen die aus Nonwoven bestehenden Schichten unmittelbar miteinander verklebt sind, und
wobei die Bahngeschwindigkeit, mit der das Laminat (12) die zweite Kaschierstation (13) durchläuft, so gesteuert wird, dass diese Bahngeschwindigkeit stets kleiner ist als die Geschwindigkeit, mit der die Materialbahnen (10, 11) aus Nonwoven und die elastischen Folienstreifen (4) in der ersten Kaschierstation (9) während der Laminatbildung zusammengeführt werden.

2. Verfahren nach Anspruch 1, wobei in der zweiten Kaschierstation (13) Streifen aus Nonwoven oder Folienstreifen aus einem nicht elastischen Polymer als Verstärkungsstreifen (6) auf einer ersten Seite des Laminats (12) aufkaschiert werden.

3. Verfahren nach Anspruch 2, wobei in der zweiten Kaschierstation (13) zusätzlich Bänder (7) mit Verschlusselementen auf der zweiten Seite des Laminats (12) aufkaschiert werden.

4. Verfahren nach einem der Ansprüche 1 bis 3, wobei die außenseitig auf das Laminat (12) aufkaschierten nicht elastischen Materialstreifen (6, 7) breiter sind als die Bahnabschnitte (5) zwischen den elastischen Folienstreifen (4).

5. Verfahren nach einem der Ansprüche 1 bis 4, wobei zur Herstellung des in der ersten Kaschierstation (9) gefertigten Laminats und zum Aufkaschieren der nicht elastischen Materialstreifen (6, 7) in der zweiten Kaschierstation (13) Hotmelt-Klebstoffe verwendet werden.

6. Verfahren nach einem der Ansprüche 1 bis 5, wobei die Bahngeschwindigkeit in der zweiten Kaschierstation (13) und die Geschwindigkeit, mit der die Materialbahnen (10, 11) aus Nonwoven und die elastischen Folienstreifen (4) in der ersten Kaschierstation (4) zusammengeführt werden, so eingestellt werden, dass die Geschwindigkeiten in einem vorgegebenen konstanten Verhältnis stehen.

7. Verfahren nach Anspruch 6, wobei die Bahngeschwindigkeit in der zweiten Kaschierstation (13) um 0,1 bis 1,0 % von der Geschwindigkeit, mit der die Materialbahnen (10, 11) aus Nonwoven und die elastischen Folienstreifen (4) in der ersten Kaschierstation (9) zusammengeführt werden, abweicht.

## Claims

1. A method for producing a material web from which elastically extensible nappy closure elements can be stamped,
wherein parallel film strips (4) which are spaced apart from one another and made of an elastically extensible polymer are laminated in a first laminating station (9) between two material webs (10, 11) of nonwoven and the web sections (5) are adhesively bonded to one another between the elastic film strips (4),
wherein the laminate (12) produced in the first laminating station (9) is supplied to a second laminating station (13) in which tension-proof, non-elastic material strips (6, 7) are laminated onto the laminate (12) on the outside,
wherein the non-elastic material strips (6, 7) are applied to web sections of the laminate (12) in which the layers consisting of nonwoven are adhesively bonded directly to one another and
wherein the web speed at which the laminate (12) passes through the second laminating station (13) is controlled such that this web speed is always lower than the speed at which the material webs (10, 11) of nonwoven and the elastic film strips (4) are brought together in the first laminating station (9) during the laminate formation.

2. The method according to claim 1, wherein in the second laminating station (13) strips of nonwoven or film strips of a non-elastic polymer are laminated as reinforcing strips (6) on a first side of the laminate (12).

3. The method according to claim 2, wherein in the second laminating station (13) bands (7) with closure elements are additionally laminated on the second side of the laminate (12).

4. The method according to any one of claims 1 to 3, wherein the non-elastic material strips (6, 7) laminated onto the laminate (12) on the outside are broader than the web sections (5) between the elastic film strips (4).

5. The method according to any one of claims 1 to 4, wherein hot-melt adhesives are used to produce the laminate fabricated in the first laminating station (9) and to laminate the non-elastic material strips (6, 7) in the second laminating station (13).

6. The method according to any one of claims 1 to 5, wherein the web speed in the second laminating station (13) and the speed at which the material webs (10, 11) of nonwoven and the elastic film strips (4) are brought together in the first laminating station (9) are adjusted so that the speeds are in a predetermined constant ratio.

7. The method according to claim 6, wherein the web speed in the second laminating station (13) differs by 0.1 to 1.0% from the speed at which the material webs (10, 11) of nonwoven and the elastic film strips (4) are brought together in the first laminating station (9).

## Revendications

1. Procédé de fabrication d'une bande de matériau, dans laquelle on peut découper des éléments de fermeture de couche extensibles élastiquement,
des bandes de film parallèles et espacées les unes des autres (4) en polymère extensible élastiquement étant laminées dans une première station de laminage (9) entre deux bandes de matériau (10, 11) en non-tissé et les sections de bande (5) étant collées ensemble entre les bandes de film élastiques (4),
le laminé (12) fabriqué dans la première station de laminage (9) étant acheminé à une deuxième station de laminage (13) dans laquelle des bandes de matériau non élastiques et résistantes à la traction (6, 7) sont contrecollées sur l'extérieur du laminé (12),
les bandes de matériau non élastiques (6, 7) étant appliquées sur des sections de bande du laminé (12) dans lesquelles les couches composées de non-tissé sont collées directement ensemble, et
la vitesse de la bande à laquelle le laminé (12) traverse la deuxième station de laminage (13) est commandée de manière à ce que cette vitesse de bande soit toujours inférieure à la vitesse à laquelle les bandes de matériau (10, 11) en non-tissé et les bandes de film élastiques (4) sont rassemblées dans la première station de laminage (9) pendant la formation du laminé.

2. Procédé selon la revendication 1, dans lequel, dans la deuxième station de laminage (13), des bandes de non-tissé ou des bandes de film en polymère non élastique sont contrecollées sous forme de bandes de renfort (6) sur une première face du laminé (12).

3. Procédé selon la revendication 2, dans lequel, dans la deuxième station de laminage (13), des bandes (7) à éléments de fermeture sont en plus contrecollées sur la deuxième face du laminé (12).

4. Procédé selon une des revendications 1 à 3, dans lequel les bandes de matériau non élastiques (6, 7) contrecollées sur l'extérieur du laminé (12) sont plus larges que les sections de bande (5) entre les bandes de film élastiques (4).

5. Procédé selon une des revendications 1 à 4, dans lequel, pour fabriquer le laminé fabriqué dans la première station de laminage (9) et pour contrecoller la bande de matériau non élastique (6, 7) dans la deuxième station de laminage (13), des colles thermofusibles sont utilisées.

6. Procédé selon une des revendications 1 à 5, dans lequel la vitesse de la bande dans la deuxième station de laminage (13) et la vitesse à laquelle les bandes de matériau (10, 11) en non-tissé et les bandes de film élastiques (4) sont rassemblées dans la première station de laminage (9) sont réglées de manière à ce que ces vitesses soient en rapport prédéfini constant.

7. Procédé selon la revendication 6, dans lequel la vitesse dans la deuxième station de laminage (13) diffère de 0,1 à 10 % de la vitesse à laquelle les bandes de matériau (10, 11) en non-tissé et les bandes de film élastiques (4) sont rassemblées dans la première station de laminage (9).
